# EUROPEAN PATENT SPECIFICATION

(11) **EP 0 968 736 B1**
(45) Date of publication and mention of the grant of the patent: **14.11.2001**
(21) Application number: 98850117.7
(22) Date of filing: 03.07.1998
(51) Int. Cl.: A61M 39/02

(54) **Medical Valve**
Medizinisches Ventil
Clapet médical

(43) Date of publication of application: 05.01.2000
(73) Proprietor: Becton Dickinson Infusion Therapy AB, 25106 Helsingborg (SE)
(72) Inventor: Österlind, Roland Jörgen, 263 54 Höganäs (SE)
(74) Representative: Bengtsson, Peggy Katrin

(56) References cited:
- EP-A- 0 224 641
- US-A- 3 416 567
- US-A- 3 906 930
- US-A- 5 098 410

## Description

### Technical field of the Invention

The present invention relates to a syringe valve for feeding of at least one fluid which is to be injected into the body, comprising a housing which constitutes a chamber having an outlet and an injection port for supplying the fluid, wherein a tubular resilient member is positioned in the housing in initial sealing contact with the injection port.

### Background art

Syringe valves are used for supplying medication directly to the vein of a patient. They are also used for supplying supplementary medication during the parental administration of for example infusion solutions. In such case, the valve is usually provided between an infusion apparatus containing a reservoir of an infusion solution, and a catheter or steel needle, of which one end is inserted in the vein of the patient.

A syringe valve is disclosed in US-3 416 567. This valve comprises a valve housing having an inlet orifice, an outlet orifice and a passageway of circular cross-section, extending between the inlet orifice and the outlet orifice. The housing is provided with a feed-in orifice opening to the passageway, said opening serving as an inlet for the supplementary medication. In the housing there is further provided a resilient, tubular insert which is positioned in the passageway so as to seal the feed-in orifice. To supply a medicament through the feed-in orifice, a syringe without needle containing the medicine is applied at the feed-in orifice. The medical fluid is pressed out of the syringe, and exert a pressure on the resilient, tubular insert. At a certain fluid pressure, the resilient insert will collapse, and thus open the valve, so that the supplementary medicament can flow into the passageway and mix with the infusion fluid.

This valve have however several disadvantages. The opening pressure of the valve is depending on the length of the tubular insert as well as its position inside the housing. Considerable fluctuations in the opening pressure between different valve units will thus occur. Especially, unusually high opening pressures cause extra problems, since correctly dosing medicine is difficult as the pressure quickly decreases after the opening instant. A risk of overdosing is then at hand.

One problem with the present valves is the small acceptable tolerance interval, which result in a difficult manufacturing problem, which would be very expensive if possible to solve. An additional problem is the precise positioning of the tubular insert inside the housing.

Another problem is that the very first opening of the valve requires an even higher pressure than subsequent openings. This is probably due to effects from handling or manufacturing, and the phenomenon is particularly pronounced for valves that have been submitted to sterilization radiation.

Another syringe valve of the type mentioned in the preamble is disclosed in US 3 906 930. This document discloses a valve comprising a tube means. The housing of the valve is provided with a step, which prevents the tube means from being further inserted in the housing.

Yet another syringe valve is disclosed in EP 0 224 641. This document discloses a valve in which housing the placement of the tube member is guided by an indentation in which the tube means is to be provided.

However, the functions of both these valves are dependent on the length of the tubular insert as well as its position inside the housing.

### Summary of the invention

It is therefore the subject of this invention to provide a syringe valve with a relatively small opening pressure, which can be held more constant for valve units of the same type. The pressure difference between the first opening of the valve and the subsequent openings should also be reduced.

This is provided by a syringe valve according to the features of claim 1, in which the inner wall of the housing is provided with means for shortening the sealing contact length between the inner wall and the resilient member by creating an open space between the tubular resilient member and the housing, said sealing contact length determining the opening pressure of the valve.

Thus the sealing contact length, that is the shortest length of sealing contact between the housing and the resilient member, is made shorter than in prior art valves. A shorter sealing length diminishes the pressure needed to open the valve and also provides a simple way of controlling it. The length and positioning of the tubular member is no longer of importance for the opening pressure. The tolerances of the corresponding measures in manufacture can thus be widened, and the cost of these reduced.

It has also been found that the short sealing length reduces the problem with a considerably higher first opening pressure at the first opening occasion. This could be due to that an adhesiveness between the housing and the tubular member affects a smaller area as the sealing length decreases.

The shortest sealing length can advantageously be defined by an indentation in the valve housing which creates an opening between the inserted tubular member and the housing in an area between the sealing length and one end of the tubular member.

A housing with such an indentation is easily manufactured without expensive changes in the production procedure.

If at least two fluids are to be injected to the body, one fluid could be supplied to the chamber through the injection port and the other fluid could be supplied to the chamber through an inlet.

### Brief description of the drawings

Fig 1 is a cross-sectional view or an embodiment of a valve according to the invention.
Fig 2 is an enlarged view of a part of fig 1.
Fig 3 is a cross-sectional view at the line III-III of fig 1.
Fig 4 is a cross-sectional view or another embodiment of a valve according to the invention.
Fig 5 is an enlarged view of a part of fig 4.
Fig 6 is a cross-sectional view at the line VI-VI of fig 4.

### Detailed description of preferred embodiments of the Invention

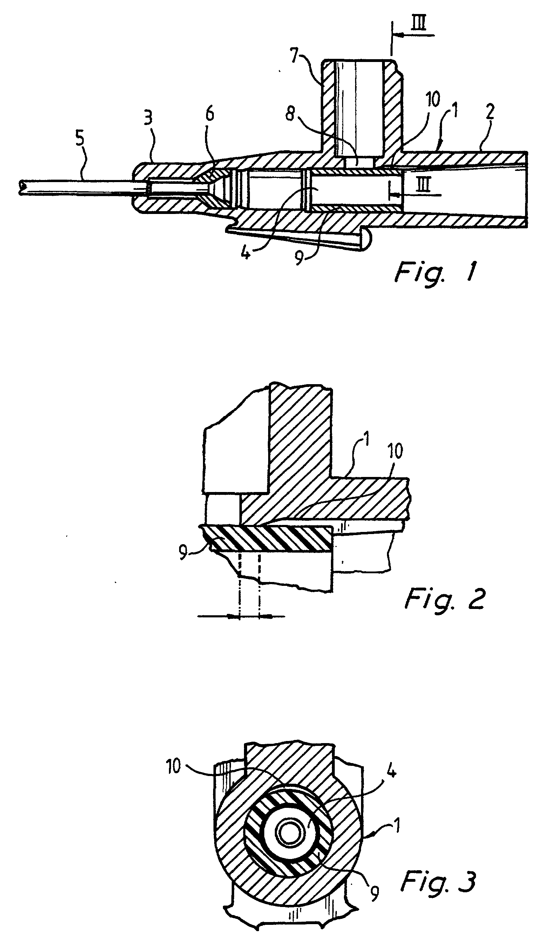

Fig 1 discloses an embodiment of a syringe valve according to the invention. The valve housing 1 has an inlet end 2 and an outlet end 3, connected by a passageway 4, which circumference, in this case, is circular. The outlet end 3 is designed to sealingly contain the end of a catheter 5, which is held in place by a bushing 6. The inlet end 2 could for example be connected to an infusion apparatus, for simultaneous feeding of two fluids into the body. It could also be sealed, by for example a plug, if only one fluid is to be injected to the body.

The housing 1 further comprises a sleeve 7 for supplying medicaments, which is designed to hold a syringe, for example a Luer-syringe. Said sleeve 7 opens to an injection port 8 in the passageway. If the inlet end 2 is connected to an infusion apparatus, the passageway 4 of the housing thus also serves as a mixing chamber for the infusion fluid flowing from the inlet 2 to the outlet 3 and a supplementary medicament supplied through the injection port 8.

In the chamber 4 a tubular resilient valve member 9 is positioned in such way that the injection port 8 is separated from the passageway 4. Towards the inlet end 2, adjacent to the injection port 8, the inner wall of the housing 1 is provided with an indentation 10, which creates an open space between the tubular valve member 9 and the housing 1 next to the injection port 8. The length of the housing 1 located between this space and the injection port 8 defines the sealing length of the valve. This sealing length is preferably in the interval 0,3 mm - 3 mm, such as 0,8 mm.

From fig 3 it is clearly seen that the indentation 10 in this case is located only at a part of the inner wall of the housing 1 next to the injection port 8.

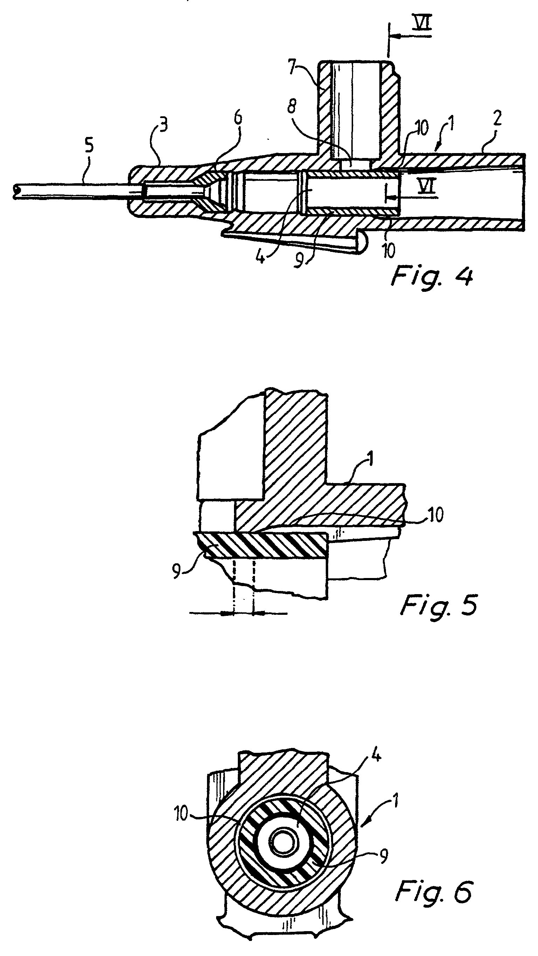

The embodiment of fig 4-6 differs from the one in fig 1-3 in that the indentation 10 extends around the whole of the inner wall of the housing 1 next to the injection port 8.

When a medicament is supplied from the injection port 8, the resilient member 9 respond to the pressure from above and opens the injection port 8. The medication then flows into the chamber 4 and is transported to the patients body through the outlet 3. As soon as the injection pressure ceases, the resilient valve member 9 is again brought into sealing relation with the injection port 8, as a result of its own resiliency. When an infusion solution is present in the chamber 4, the valve member 9 is brought into its sealing position also by the outwardly exerted pressure of the flowing infusion solution.

Many other embodiments than those described above are within the scope of the present invention. For example, the housing of the valve can have a different design and geometry. For the supply of only one fluid to the patient, the housing can be made without an inlet 2. The shortest sealing length can be achieved by at least one protrusion instead of an indentation in the housing. The cross-section of the chamber is preferably circular, but could have other shapes. The design of the resilient tubular member must of course be adjusted to the shape of the housing. The means for shortening the sealing contact length can be provided towards the inlet end instead of the outlet end of the housing.

## Claims

1. A syringe valve for feeding of at least one fluid which is to be injected into the body, comprising a housing (1) which constitutes a chamber (4) having an outlet (3) and an injection port (8) for supplying the fluid, wherein a tubular resilient member (9) is positioned in the housing (1) in initial sealing contact with the inner wall of the housing (1) around the injection port (8), **characterised in that** said inner wall is provided with means (10) for shortening the sealing contact length between the inner wall and the resilient member (9) by creating an open space between the tubular resilient valve member (9) and the housing (1), said sealing contact length determining the opening pressure of the valve.

2. A syringe valve according to claim 1, **characterised in** an inlet (2) being provided so that at least two fluids are injectable to the body, one fluid being supplied to the chamber through the injection port (8) and the other fluid being supplied to the chamber (4) through an inlet (2).

3. A syringe valve according to claim 1-2, **characterised in** that said means (10) extends only along a part of the inner wall of the housing (1) around the injection port (8).

4. A syringe valve according to claim 1-2, **characterised in** that said means (10) extends around the whole of the inner wall of the housing (1).

5. A syringe valve according to claim 1-4, **characterised in** that said means (10) is at least one indentation in the inner wall of the housing (1).

6. A syringe valve according to claim 1-5, **characterised in** that said means (10) is at least one protrusion in the inner wall of the housing (1).

## Patentansprüche

1. Spritzenventil zum Zuführen mindestens einer in den Körper zu injizierenden Flüssigkeit, umfassend ein Gehäuse (1), das eine Kammer (4) mit einem Auslass (3) und einem Injektionsport (8) zur Abgabe der Flüssigkeit darstellt, worin ein schlauchförmiges nachgiebiges Element (9) im Gehäuse (1) in anfänglich dichtendem Kontakt mit der Innenwand des Gehäuses (1) um den Injektionsport (8) angeordnet ist, **dadurch gekennzeichnet, dass** diese Innenwand mit einem Mittel (10) zum Verkürzen der Dichtkontaktlänge zwischen der Innenwand und dem nachgiebigen Element (9) durch Schaffung eines offenen Raums zwischen dem schlauchförmigen nachgiebigen Ventilelement (9) und dem Gehäuse (1) versehen ist, wobei diese Dichtkontaktlänge den Öffnungsdruck des Ventils bestimmt.

2. Spritzenventil nach Anspruch 1, **dadurch gekennzeichnet, dass** ein Einlass (2) vorgesehen ist, so dass mindestens zwei Flüssigkeiten in den Körper injiziert werden können, wobei eine Flüssigkeit der Kammer durch den Injektionsport (8) und die andere Flüssigkeit der Kammer (4) durch einen Einlass (2) zugeführt wird.

3. Spritzenventil nach Anspruch 1-2, **dadurch gekennzeichnet, dass** das Mittel (10) sich nur entlang eines Teils der Innenwand des Gehäuses (1) um den Injektionsport (8) herum erstreckt.

4. Spritzenventil nach Anspruch 1-2, **dadurch gekennzeichnet, dass** das Mittel (10) sich um die gesamte Innenwand des Gehäuses (1) erstreckt.

5. Spritzenventil nach Anspruch 1-4, **dadurch gekennzeichnet, dass** das Mittel (10) mindestens eine Vertiefung in der Innenwand des Gehäuses (1) darstellt.

6. Spritzenventil nach Anspruch 1-5, **dadurch gekennzeichnet, dass** das Mittel (10) mindestens ein Vorsprung in der Innenwand des Gehäuses (1) darstellt.

## Revendications

1. Clapet pour seringue pour l'alimentation d'au moins un fluide qui doit être injecté dans le corps, comprenant un boîtier (1) qui constitue une chambre (4) possédant une sortie (3) et un orifice d'injection (8) pour alimenter le fluide, dans lequel un élément résilient tubulaire (9) est positionné dans le boîtier (1) en contact d'étanchéisation initiale avec la paroi interne du boîtier (1) autour de l'orifice d'injection (8), **caractérisé en ce que** ladite paroi interne est munie d'un moyen (10) pour raccourcir la longueur de contact d'étanchéisation entre la paroi interne et l'élément résilient (9) en créant un espace ouvert entre l'élément de clapet résilient tubulaire (9) et le boîtier (1), ladite longueur de contact d'étanchéisation déterminant la pression d'ouverture du clapet.

2. Clapet pour seringue selon la revendication 1, **caractérisé en ce qu'**une entrée (2) est prévue, de telle sorte qu'au moins deux fluides peuvent être injectés dans le corps, un fluide étant alimenté dans la chambre à travers l'orifice d'injection (8) et l'autre fluide étant alimenté dans la chambre (4) à travers l'entrée (2).

3. Clapet pour seringue selon les revendications 1-2, **caractérisé en ce que** ledit moyen (10) s'étend uniquement le long d'une partie de la paroi interne du boîtier (1) autour de l'orifice d'injection (8).

4. Clapet pour seringue selon les revendications 1-2, **caractérisé en ce que** ledit moyen (10) s'étend autour de la totalité de la paroi interne du boîtier (1).

5. Clapet pour seringue selon les revendications 1-4, **caractérisé en ce que** ledit moyen (10) représente au moins une dentelure pratiquée dans la paroi interne du boîtier (1).

6. Clapet pour seringue selon les revendications 1-5, **caractérisé en ce que** ledit moyen (10) représente au moins une saillie pratiquée dans la paroi interne du boîtier (1).
